# EUROPEAN PATENT APPLICATION

(11) **EP 3 220 300 A1**
(43) Date of publication of application: **20.09.2017**
(21) Application number: 17161120.5
(22) Date of filing: 15.03.2017
(51) Int. Cl.: G06F 19/00

(54) **SYSTEM AND METHOD FOR OBTAINING CONTEXTUAL DATA ASSOCIATED WITH TEST RESULTS FROM HEALTH MONITORING DEVICES**

(30) Priority: 16.03.2016 US 201662308868 P
(71) Applicant: CRF Inc., Plymouth Meeting, PA 19462 (US)
(72) Inventor: STROBRIDGE, Richard, Alpine, CA California 91901 (US)
(74) Representative: Berggren Oy, Helsinki & Oulu

(57) **Abstract**

The invention provides a system and method for configurable queries based on the real-time results from a plurality of health monitoring devices. The results from the queries provide context to the real-time results of the health monitoring devices are communicated back to a network to inform future questions for that patient and the patient population in general. The system of the present invention provides context based on a set or sets of queries that may inform follow up questions.

## Description

### Field of technology

The present invention relates generally to health monitoring devices and more particularly to a system and method for acquiring contextual data associated with the testing data gathered from health monitoring devices.

### Prior art

In the diagnosis and assessment of disease and subsequent patient outcomes, clinicians and clinical scientists rely on a variety of tests to gather physiological data. These tests have developed overtime to detect and measure nearly every function and physiological change in the human body. While this physiological data provides valuable insight into a patient's condition, practitioners also rely heavily on relevant contextual data to assist in the interpretation and analysis of the physiological data. The types of contextual data associated with a particular physiological measurement will vary greatly, but examples may include the level of physical activity, caloric intake, fluids consumed, and/or time of taking a medication.

Typically, in a clinical setting, the contextual data is collected by a practitioner through a set of questions and answers that may be manually entered into the patient's file at the time of the consultation or physiological test. Similarly, in a clinical trial setting, participants may be required toprovide contextual data by filling out a form that is mailed to or sent home with the study participant. While these are both viable options that are still in use today, with advancements in medical sensing technologies and remote, real-time patient monitoring, a need exists for a system capable of gathering contextual data that is based on the real-time results of health monitoring device data.

### Short description

The present invention overcomes these and other deficiencies of the prior art by providing a system and method for configurable queries based on the real-time results from a plurality of health monitoring devices. The results from the queries provide context to the real-time results of the health monitoring devices and are communicated back to a network to inform future questions for that patient and the patient population in general. The system of the present invention provides context based on a set or sets of queries that may inform follow up questions. The context and queries may also be informed based on predetermined thresholds for a plurality of health monitoring devices communicating with the system over a network.

The foregoing, and other features and advantages of the invention, will be apparent from the following, more particular description of the preferred embodiments of the invention, the accompanying drawings, and the claims.

### List of figures

For a more complete understanding of the present invention, and the advantage thereof, reference is now made to the ensuing descriptions taken in connection with the accompanying drawings briefly described as follows:
Fig. 1 illustrates an overall depiction of the system and method according to an embodiment of the invention;
Fig. 2 illustrates a process flow of sending collected data from a glucose device to the system prompting a predetermined initial set of questions to add context according to an embodiment of the invention;
Fig. 3 illustrates a process flow of sending follow up questions dependent upon whether blood glucose levels are above or below a predetermined threshold according to an embodiment of the present invention;
Figs. 4A and 4B illustrate an example of a database of the system including data collected and stored by a network of the system related to predetermined thresholds associated with test results from a health monitoring device according to an embodiment of the invention;
Fig. 5 illustrates an example of an initial questions database of the system including data collected and stored by a network of the system according to an embodiment of the invention; and
Fig. 6 illustrates an example practitioner interface for the entry of data into the various databases of the system according to an embodiment of the invention.

### Description of the invention

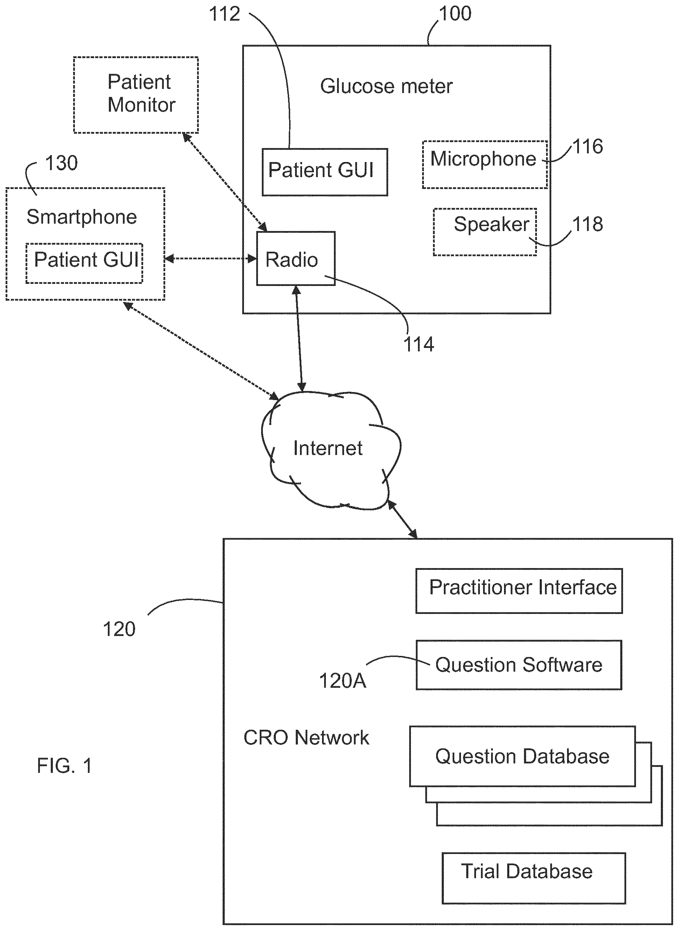

Preferred embodiments of the present invention and their advantages, as well as the operation of various embodiments of the invention are described in detail below with reference to the accompanying Figs. 1-6, wherein like reference numerals refer to like elements. The following detailed description of the system and method of the present invention describes a use case in the context of a contract research organization (CRO) conducting a clinical trial. One of ordinary skill in the art will readily appreciate that the system and methods of the present invention are not limited to the use case described herein but are readily applicable to any activity requiring continuous patient care and monitoring. For example, a clinician providing remote monitoring of a patient using health monitoring devices will benefit from the contextual data gathering capabilities of the system and method described herein.

While the system and methodology described herein is extensible to health monitoring devices generally, for ease and the sake of clarity, the use case described herein with respect to the health monitoring device in the system and method of the present invention will be described in the context of a patient using a glucose meter.

The system and method described herein is characterized by the ability to facilitate inclusion and/or connectivity with a plurality of connected devices. The connected devices may be connected to and in communication with the system via a wired connection or wireless connection. The wireless connection may be facilitated by known wireless protocols including, by way of non-limiting examples, infrared, Bluetooth, ZigBee, Wi-Fi, 3G/4G wireless protocols, radio frequency identification (RFID), and near field communication (NFC) protocols. In an embodiment, the connected devices are health monitoring devices including, but not limited to, spirometer, glucose meter, CPAP machine, indoor air quality (IAQ) meter, ventilator, pulse oximeter, sphygmomanometer, thermometer, nebulizers, heart monitors, and the like. In further embodiments of the present invention the connected devices, in addition to the health monitoring devices include, by way of non-limiting examples, smartphones, tablets, desktop computers, laptop computers, and the like.

In some embodiments, the health monitoring device includes a graphical user interface (GUI) configured to facilitate communication with the network in which the user receives a set of questions to derive contextual data associated with the data collected by the health monitoring device. In other embodiments, the communication with the network in which the user receives a set of questions to derive contextual data associated with the data collected by the health monitoring device is facilitated by way of a connected device, as described herein, such as smartphones, tablets, desktop computers, laptop computers, and the like.

Referring to Fig. 1 is an overall depiction of the system and method according to an embodiment of the invention. A health monitoring device such as a glucose meter 110 is configured to communicate with a network 120 and a connected device 130. The glucose meter 110 is configured to communicate with the network 120 of the system via a wireless connection such as, Bluetooth, ZigBee, Wi-Fi, and 3G/4G wireless protocols. The glucose meter 110 may include a GUI 112 and a radio 114 for communication with the network 120 and connected device 130. In some embodiments, the glucose meter 110 may include a microphone 116 and a speaker 118 to facilitate interactive voice response (IVR) and audible alerts. The network 120 includes a plurality of databases for storing patient health monitoring device data, historical data, and questions for gathering contextual data from the patient. The information stored on the network 120 may be accessible by the practitioner through a GUI.

The system of the present invention advantageously provides for configurable queries based on the real-time results from health monitoring devices. The results from the queries provide context to the real-time results of the health monitoring devices and are communicated back to a network to inform future questions for that patient and the patient population in general.

As an example of the application of the system within the context of a CRO conducting a clinical trial, a patient or study participant may test blood glucose levels with a glucose meter. The glucose meter is configured to send the blood glucose results to the network thereby prompting a software module to elicit responses from the patient or study participant to a set of initial questions to gather contextual data. The initial questions are stored in a database of the system and may be set by the practitioner based on predetermined thresholds for the blood glucose results. The questions are delivered to the patient through a patient GUI, which may be a touch screen on the meter or on a smartphone or with an IVR. The responses to the initial set of questions and blood glucose test results are stored in a database of the system. The practitioner defined thresholds coincide with a separate set of questions based on the blood glucose levels of the patient or study participant. For example, if the patient has low or high blood sugar a second set of questions that is dependent on the threshold set to define low or high blood sugar levels are communicated to the patient. The questions may be required or optional and may have criteria dependent follow up questions. The system of the present invention may also be configured to utilize a plurality of health monitoring devices. For example within the context of blood glucose meters, an activity monitor and/or calorie counter may be connected to the system to provide additional data.

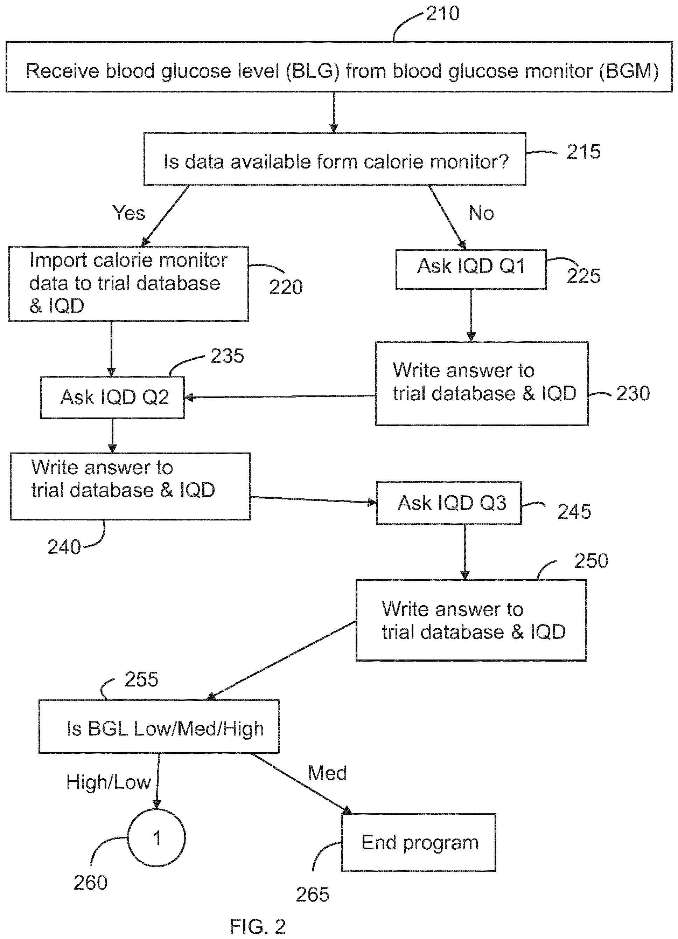

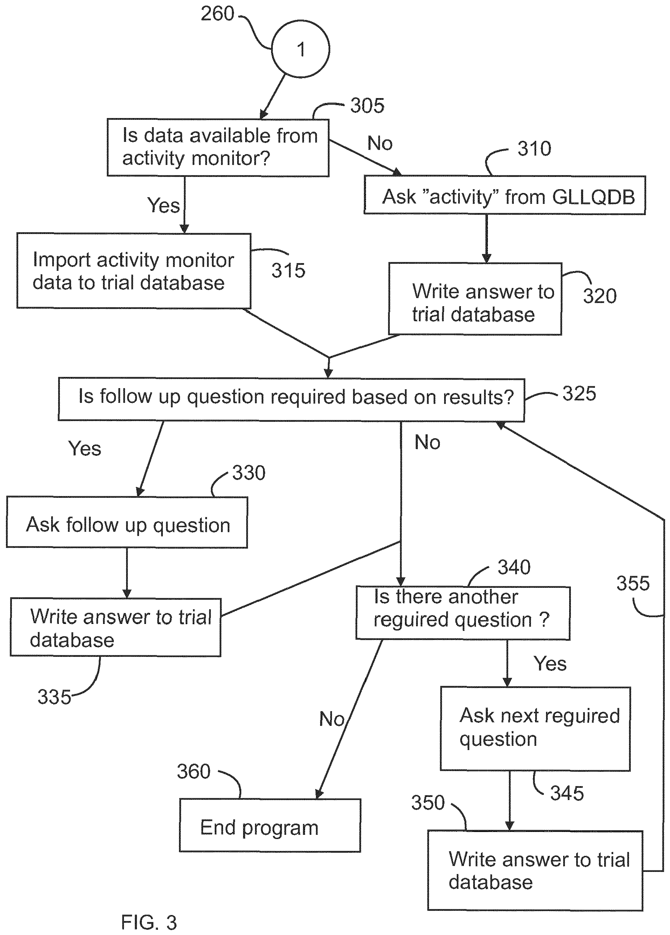

Fig. 2 illustrates a process flow of sending collected data from a glucose device to the system prompting a predetermined initial set of questions to add context according to an embodiment of the invention. At 210, the network receives test data from a glucose meter. The system then queries, at 215, whether additional data is available from another health monitoring device such as calorie meter. If an additional health monitoring device is connected, then at 220, the data from the additional health monitoring device is received and stored in a database of the system. In the example of this embodiment with respect to blood glucose data, an exemplary initial question, at 225, presented to a patient would query when the patient last ate. If the patient was using a calorie meter, then the data from that device would supply the answer to the initial question obviating the need to query when the patient last ate. Thus, in some embodiments, the system may be configured to include a plurality of connected health monitoring devices to supply data in response to a set or sets of queries from the system. The responses to the queries from the system are communicated back to the network, at 230, and stored in appropriate databases of the system to inform additional queries. Consistent with the example of this embodiment, the patient is asked, at 235, additional follow up questions by the system such as when was the last time they received insulin. The response to the query from the system is communicated back to the network, at 240, and stored in appropriate databases of the system to inform additional queries. At 245, the patient is asked an additional follow up question by the system such as when they last received medication that was either prescribed or part of the research of the clinical trial. The response to the query from the system is communicated back to the network, at 250, and stored in appropriate databases of the system to inform additional queries, if necessary. The system then, at 255, determines whether the blood glucose level of the patient is below or above a practitioner defined threshold. If the blood glucose levels of the patient are within the predetermined practitioner defined thresholds, then the program ends at 265. If the predetermined practitioner defined thresholds are exceeded, then at 260, the system continues to elicit additional information from the patient as described in Fig. 3.

Fig. 3 illustrates a process flow of sending follow up questions dependent upon whether blood glucose levels are above or below a predetermined threshold according to an embodiment of the present invention. The system of the present invention is configurable to permit practitioners to define various thresholds for any number or type of health monitoring devices and the particular test result or data received therefrom. In addition, the practitioner may include and store in a database of the system relevant questions to be communicated to the patient based upon the real-time test results from the health monitoring devices that may fall outside of the defined thresholds. If the predetermined practitioner defined thresholds are exceeded, then the system will elicit responses from the patient to add context to the test results that are above or below the defined thresholds.

For example, in the exemplary embodiment of Fig. 3, if a patient's blood glucose level is above or below the practitioner defined threshold, then a set of follow-on questions is prompted. The follow-on questions have been predetermined by the practitioner and stored in database of the system. In the example of Fig. 3, at 305µ, the first question set by the practitioner is the patient's level of activity, and therefore the system will first poll for the availability of an activity monitor. If, at 310, no activity monitor is connected to the system from which relevant data may be collected, then a question regarding the activity level of the patient is communicated to the patient. The response to the query from the system is communicated back to the network, at 320, and stored in appropriate databases of the system to inform additional queries. If an activity monitor is connected to the system, then at 315, data from the activity monitor is received by the system and stored in the appropriate databases. Dependent upon the particularities of the patient's condition or the research study protocol, at 325, an additional query may be required based on the results of the response or data associated with the first query. If the practitioner defined additional queries based on certain responses or data received with the first query, then at 330, the additional follow up question is communicated to the patient. The response to the query from the system is communicated back to the network, at 335, and stored in appropriate databases of the system to inform additional queries, if required. The system continues to determine at 340 whether additional follow up questions are required as defined by the practitioner. If a follow up question has been defined by the practitioner, then at 345, the next required question is communicated to the patient, and the response received by the system and stored in the appropriate databases at 350. The process of determining if the response to a query prompts follow up queries continues at 355 until all practitioner defined questions have been communicated to the patient. When all practitioner defined questions have been communicated to the patient, the process ends at 360.

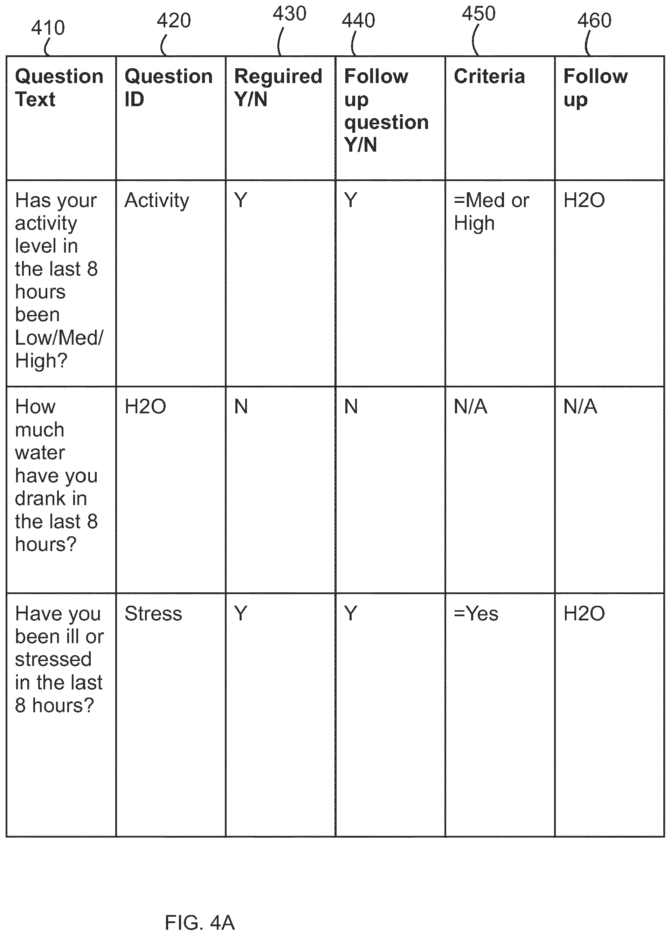

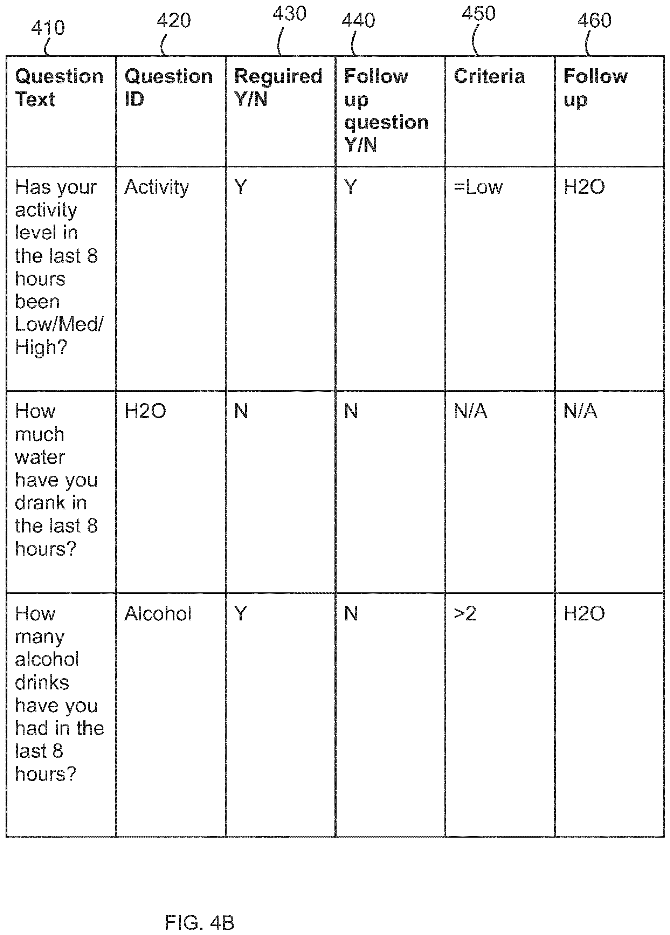

Figs. 4A and 4B illustrate an example of a database of the system including data collected and stored by a network of the system related to predetermined thresholds associated with test results from a health monitoring device according to an embodiment of the invention. The system of the present invention includes a plurality of databases associated with the network. The databases may include the question text 410, question identification (ID) 420, required designation 430, follow up question designation 440, follow up question criteria 450, and follow up question ID 460. The question text 410 is the text of the question as defined by the practitioner in the practitioner interface. The question ID 420 contains the question ID that is used by the question software and the follow up designation column of this database. The required designation 430 is a yes/no designation and defines whether a question is required by the practitioner or not. The follow up question designation 440 is a yes/no designation and defines whether a question requires a follow up question or not, as designated by the practitioner. The follow up question criteria 450 is the range of responses that if given by the patient will prompt a follow up question. The follow up question ID 460 is the question ID of the follow up question the practitioner wants asked when the patient's answer to the required question meets the criteria at 450. The process of queries defined by the practitioner may continue with a chain of follow up questions, if so designated by the practitioner.

It is to be understood that the content of the plurality of databases of the system associated with practitioner defined queries will differ depending upon what the particular database is related to. For example, when the system receives a test result or data from a health monitoring device, an initial query or queries is or are prompted based on the test result or data. The initial query or queries is based upon the real-time data received from the health monitoring data and may be defined by the practitioner and stored in a database of initial queries. If the test result or data from the health monitoring device falls below or exceeds a practitioner defined threshold, then a database of follow up questions relevant for that condition is prompted. For example, if the test result or data falls below the practitioner defined threshold, then queries relevant for that condition are prompted as described with respect to Figs. 4A and 4B herein. If the test result or data exceeds the practitioner defined threshold, then a separate set of queries relevant for that condition are prompted as described with respect to Figs. 4A and 4B herein. Thus, a practitioner or principal investigator may designate a threshold for a particular health monitoring device and define a set of queries to be store in a database of the system to gather contextual data relevant for a patient condition that may be within, fall below, or exceed the practitioner defined thresholds.

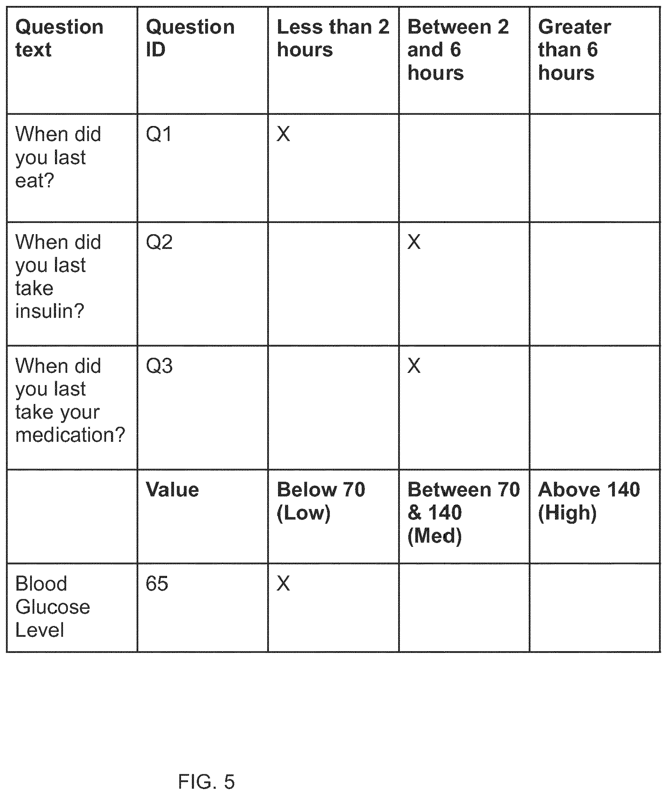

Fig. 5 illustrates an example of an initial questions database of the system including data collected and stored by a network of the system according to an embodiment of the invention. The system of the present invention includes a plurality of databases associated with the network. The initial questions database may include the question text 510, question ID 520, the low value 530, medium range 540 and high value 550. The question text 510 is defined by the practitioner in the practitioner interface. The question ID 520 is used by a software module of the system. The low value 530, medium range 540 and high value 550 hold a marker for the patient's response to each of the initial questions, which in this example are determining when the patient last ate, took insulin, and took the medication that is the subject of this research or prescribed by the practitioner. In the embodiment of Fig. 5, the practitioner set the low threshold at less than 2 hours and the high threshold at greater than 6 hours. In this non-limiting embodiment for purposes of example only, the patient ate in the last two hours and received insulin and their medication between 2 and 6 hours. In the last row of the database of this non-limiting example, the results of the glucose test are stored as both a numerical value and its place relative to the high and low limits set by the practitioner.

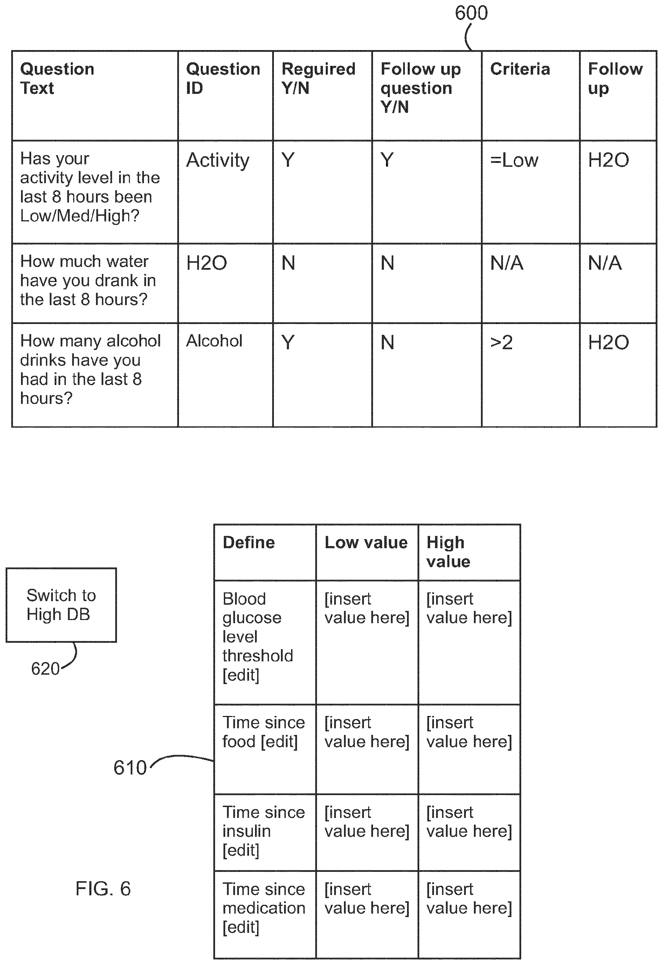

Fig. 6 illustrates an example practitioner interface for the entry of data into the various databases of the system according to an embodiment of the invention. The practitioner interface is accessed through the network. The interface allows the practitioner to define patient queries and threshold values for the required questions and the connected health monitoring devices that will prompt relevant follow up queries for a patient condition that may be within, fall below, or exceed the practitioner defined thresholds. As a non-limiting example, 610 illustrates a practitioner interface window for defining various entries to be stored in the databases of the system and used by the system to inform relevant future follow up queries for a patient. 600 illustrates a non-limiting example of a practitioner interface for defining the required and follow up high/low glucose level questions. By way of non-limiting example, the practitioner may be able to define if the required questions require a follow up question, what criteria would prompt a follow up question, and what that follow up question's question ID is. The practitioner interface may also include a radio button 620 enabling the practitioner to switch between the various databases of the system to further define relevant thresholds and queries.

It is to be understood that the databases described herein are configurable and applicable to any number of health monitoring devices and disease states. One of ordinary skill in the art readily appreciates and understands the implementation of the system described herein to any situation where a practitioner or clinical scientist, or the like needs to monitor a chronic care patient such as in a practitioner's ongoing patient care, whether remote or not, or principal investigator monitoring patient's in a clinical trial.

Those of skill in the art will appreciate that the various illustrative logical blocks, modules, units, and algorithm steps described in connection with the embodiments disclosed herein can often be implemented as electronic hardware, computer software, or combinations of both. To clearly illustrate this interchangeability of hardware and software, various illustrative components, blocks, modules, and steps have been described above generally in terms of their functionality. Whether such functionality is implemented as hardware or software depends upon the particular constraints imposed on the overall system. Skilled persons can implement the described functionality in varying ways for each particular system, but such implementation decisions should not be interpreted as causing a departure from the scope of the invention. In addition, the grouping of functions within a unit, module, block, or step is for ease of description. Specific functions or steps can be moved from one unit, module, or block without departing from the invention.

The various illustrative logical blocks, units, steps and modules described in connection with the embodiments disclosed herein, and those provided in the accompanying documents, can be implemented or performed with a processor, such as a general purpose processor, a digital signal processor (DSP), an application specific integrated circuit (ASIC), a field programmable gate array (FPGA) or other programmable logic device, discrete gate or transistor logic, discrete hardware components, or any combination thereof designed to perform the functions described herein, and those provided in the accompanying documents. A general-purpose processor can be a microprocessor, but in the alternative, the processor can be any processor, controller, microcontroller, or state machine. A processor can also be implemented as a combination of computing devices, for example, a combination of a DSP and a microprocessor, a plurality of microprocessors, one or more microprocessors in conjunction with a DSP core, or any other such configuration.

The steps of a method or algorithm and the processes of a block or module described in connection with the embodiments disclosed herein, and those provided in the accompanying documents, can be embodied directly in hardware, in a software module executed by a processor, or in a combination of the two. A software module can reside in RAM memory, flash memory, ROM memory, EPROM memory, EEPROM memory, registers, hard disk, a removable disk, a CD-ROM, or any other form of storage medium. An exemplary storage medium can be coupled to the processor such that the processor can read information from, and write information to, the storage medium. In the alternative, the storage medium can be integral to the processor. The processor and the storage medium can reside in an ASIC. Additionally, device, blocks, or modules that are described as coupled may be coupled via intermediary device, blocks, or modules. Similarly, a first device may be described a transmitting data to (or receiving from) a second device when there are intermediary devices that couple the first and second device and also when the first device is unaware of the ultimate destination of the data.

The above description of the disclosed embodiments, and that provided in the accompanying documents, is provided to enable any person skilled in the art to make or use the invention. Various modifications to these embodiments will be readily apparent to those skilled in the art, and the generic principles described herein, and in the accompanying documents, can be applied to other embodiments without departing from the spirit or scope of the invention. Thus, it is to be understood that the description and drawings presented herein, and presented in the accompanying documents, represent particular aspects and embodiments of the invention and are therefore representative examples of the subject matter that is broadly contemplated by the present invention. It is further understood that the scope of the present invention fully encompasses other embodiments that are, or may become, obvious to those skilled in the art and that the scope of the present invention is accordingly not limited by the descriptions presented herein, or by the descriptions presented in the accompanying documents.

## Claims

1. A system for obtaining contextual data associated with test results from at least one health monitoring device, the system comprising at least one health monitoring device to provide test results, **characterised in that** the system further comprises a practitioner interface to define questions, and at least one threshold value for the at least one health monitoring device,
and at least one database to store the test results, the questions, the at least one threshold value, and contextual data,
the practitioner interface being in communication with at least one database, and at least one database being in communication with at least one health monitoring device, and being arranged, in response to the test results of one of said health monitoring device,
to poll the other test results from the other connected health monitoring device,
to prompt a set or sets of questions being associated with the test results,
to deliver the set or sets of questions to a patient device in order to obtain said contextual data, and
to receive responses to the set or sets of questions, and store them in at least one database.

2. A system according to claim 1, **characterised in that** said test results are real-time test results.

3. A system according to claim 1 or 2, **characterised in that** the set or sets of questions comprises initial question/s and follow up question/s, the initial question/s being communicated first and the follow-up question/s after the initial question/s,
the follow-up questions being communicated when meeting follow-up question specific criteria.

4. A method for obtaining contextual data associated with test results from at least one health monitoring device, **characterised in that** the method comprises steps
to receive the test results of one of said health monitoring device,
to poll other test results from the other connected health monitoring devices,
to prompt a set or sets of questions being associated with the test results,
to deliver the set or sets of questions to a patient/s in order to obtain said contextual data,
to receive responses to the set or sets of questions, and store them.

5. A method according to claim 4, **characterised in that** said test results are real-time test results.

6. A method according to claim 4 or 5, **characterised in that** the set or sets of questions comprises initial question/s and follow up question/s, the delivering step comprising substeps
to communicate the initial question/s first and,
to communicate the follow-up question/s after the initial question/s, the follow-up questions being communicated when meeting follow-up question specific criteria.

7. A method according to claim 6, **characterised in that** said follow-up question specific criteria comprises threshold values, and certain answers to previous questions.

8. A method according to claim 7, **characterised in that** the follow-up questions provide a chain of the follow-up questions, which are communicated in turn.

9. A method according to any of claim 4 - 8, **characterised in that** method further comprises a step to predetermine questions, and threshold values.

10. A method according to any of claim 4 - 9, **characterised in that** the method is implemented with at least one processor.

11. A method according to any of claim 4 - 10, **characterised in that** at least some of said steps are arranged to run in a graphical user interface of the health monitoring device, a smartphone, tablet, desktop, computer and/or laptop computers in order to deliver the questions to the patient.

12. A method according to claim 4 - 17, **characterised in that** the health monitoring devices comprises a spirometer, glucose meter, CPAP machine, indoor air quality meter, ventilator, pulse oximeter, sphygmomanometer, thermometer, nebulizer and heart monitor.

13. A method according to any of claim 4 - 17, **characterised in that** at least some of said steps are arranged to run in the practitioner interface, which is a graphical user interface.

14. A storage medium of software arranged to be with at least one processor, **characterised in that** the storage medium of the software is arranged to provide steps for obtaining contextual data associated with test results from at least one health monitoring device, said steps comprising steps
to receive the test results of one of said health monitoring device,
to poll other test results from other connected health monitoring devices
to prompt a set or sets of questions being associated with the test results,
to deliver the set or sets of questions to a patient/s in order to obtain said contextual data,
to receive responses to the set or sets of questions, and store them.

15. A storage medium according to claim 14, **characterised in that** said test results are real-time test results.

16. A storage medium according to claim 14 or 15, **characterised in that** the set or sets of questions comprises initial question/s and follow up question/s, the delivering step comprising substeps
to communicate the initial question/s first and,
to communicate the follow-up question/s after the initial question/s, the follow-up questions being communicated when meeting follow-up question specific criteria.

17. A storage medium according to claim 16, **characterised in that** said follow-up question specific criteria comprises threshold values, and certain answers to previous questions.

18. A storage medium according to claim 17, **characterised in that** the follow-up questions provide a chain of the follow-up questions, which are communicated in turn.

19. A storage medium according to any of claim 14 - 18, **characterised in that** the steps further comprises a step to predetermine questions, and threshold values.

20. A patient device being a health monitoring device, smartphone, tablet, desktop computer, laptop computer, and the like, **characterised in that** the patient device is arranged to be a part of a system according to any of claim 1 - 3.

21. A practitioner interface being a graphical user interface, **characterised in that** the practitioner interface is arranged to be a part of a system according to claim 1 - 3.

22. A practitioner interface according to claim 22, **characterised in that** said practitioner interface is implemented as electronic hardware, computer software, or combinations of both.
